(19) Europäisches Patentamt
European Patent Office
Office européen des brevets

(11) **EP 4 606 297 A1**

(12) **EUROPEAN PATENT APPLICATION**

(43) Date of publication:
**27.08.2025 Bulletin 2025/35**

(21) Application number: 24158668.4

(22) Date of filing: **20.02.2024**

(51) International Patent Classification (IPC):
**A61B 5/00** $^{(2006.01)}$    **A61B 5/024** $^{(2006.01)}$

(52) Cooperative Patent Classification (CPC):
**A61B 5/7485; A61B 5/02416; A61B 5/7253**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Koninklijke Philips N.V.
5656 AG Eindhoven (NL)**

(72) Inventors:
- **DEN BRINKER, Albertus Cornelis
  Eindhoven (NL)**
- **DER SARKISSIAN, Henri Henroutune
  Eindhoven (NL)**
- **WUELBERN, Jan Hendrik
  5656AG Eindhoven (NL)**
- **PADALKO, Mikhail
  Eindhoven (NL)**

(74) Representative: **Philips Intellectual Property &
Standards
High Tech Campus 52
5656 AG Eindhoven (NL)**

(54) **PHOTOPLETHYSMOGRAPHY REGION OF INTEREST DETERMINATION**

(57)    Disclosed herein is a computational system (400) for determining a region of interest (414) for extracting a photoplethysmography signal. Execution of machine executable instructions (410) causes the computational system (400) to receive a sequence of optical images (412); divide a respective image region into a plurality of subsets; determine for at least some of the subsets dynamic characteristics of optical signals ($x_0$) described by intrinsic mode functions ($y_i$); cluster the plurality of subsets; select a cluster comprising a cluster characteristic satisfying a pre-defined selection criterium; and identify the pixels comprised by the selected cluster as the region of interest (414) of the optical images for extracting the photoplethysmography signal.

Fig. 1

## Description

### FIELD OF THE INVENTION

The present invention relates to the field of image processing, in particular to determining a region of interest for extracting a photoplethysmography signal of a subject within optical images.

### BACKGROUND OF THE INVENTION

Photoplethysmography (PPG) is a technique that uses changes in light interaction, e.g. light absorption, to measure blood volume changes in the microvasculature. For example, a simple PPG sensor may consist of a light source to illuminate living tissue, such as the skin, mucous membranes, and other translucent tissues, and an optical sensor, such as a photodiode, phototransistor and/or photomultiplier tube, to measure the amount of light that is scattered and/or absorbed by the tissue. The optical signal acquired by the optical sensor, i.e., the PPG signal, is indicative of blood volume changes in the microvasculature, and thus provides a noninvasive way to measure blood volume changes in (near) real-time. PPG has a wide range of applications.

### SUMMARY OF THE INVENTION

The invention provides for a computational system, a medical imaging system, a method, and a computer program in the independent claims. Embodiments are given in the dependent claims.

In one aspect the invention provides for a computational system for determining a region of interest for extracting a photoplethysmography signal of a subject within optical images. The computational system comprises a processor and a memory storing machine executable instructions. An execution of the machine executable instructions using the processor causes the computational system to receive a sequence of optical images. The optical images comprise optical imaging data of at least a part of a tissue of interest of the subject, which is of interest for the photoplethysmography signal extraction. Execution of the machine-executable instructions further causes the computational system, for the sequence of optical images, to divide a respective image region comprised by each optical image of the sequence into a plurality of subsets of pixels. Execution of the machine-executable instructions further causes the computational system to determine for at least some of the subsets dynamic characteristics of optical signals comprised by the respective subsets. Each of the dynamic characteristics is described by an intrinsic mode function. The respective intrinsic mode function is indicative of a temporal signal dynamic of the optical signal within the respective subset over the sequence of optical images.

Execution of the machine-executable instructions further causes the computational system to cluster the plurality of subsets, using the determined dynamic characteristics, to group the subsets of pixels into clusters with similar signal dynamics. Execution of the machine-executable instructions further causes the computational system to select a cluster from the resulting clusters comprising a cluster characteristic satisfying a pre-defined selection criterium. Execution of the machine-executable instructions further causes the computational system to identify the pixels comprised by the selected cluster as the region of interest of the optical images for extracting the photoplethysmography signal of the subject.

In another aspect the invention provides for a medical imaging system. The medical imaging system comprises a medical sensor system configured for acquiring medical imaging data of a subject, an optical sensor configured for acquiring optical images of the subject, and the computational system for determining a region of interest for extracting a photoplethysmography signal of a subject within optical images.

In another aspect the invention provides for a method for determining a region of interest for extracting a photoplethysmography signal of a subject within optical images. The method comprises receiving a sequence of optical images. The optical images comprise optical imaging data of at least a part of a tissue of interest of the subject, which is of interest for the photoplethysmography signal extraction. The method further comprises, for the sequence of optical images, dividing a respective image region comprised by each optical image of the sequence into a plurality of subsets of pixels. The method further comprises determining for at least some of the subsets dynamic characteristics of optical signals comprised by the respective subsets. Each of the dynamic characteristics is described by an intrinsic mode function. The respective intrinsic mode function is indicative of a temporal signal dynamic of the optical signal within the respective subset over the sequence of optical images.

The method further comprises clustering the plurality of subsets, using the determined dynamic characteristics, to group the subsets of pixels into clusters with similar signal dynamics. The method further selecting a cluster from the resulting clusters comprising a cluster characteristic satisfying a pre-defined criterium. The method further comprises identifying the pixels comprised by the selected cluster as the region of interest of the optical images for extracting the photoplethysmography signal of the subject.

In another aspect the invention provides for a computer program comprising machine executable instructions for determining a region of interest for extracting a photoplethysmography signal of a subject within optical images. An execution of the machine executable instructions using a processor of a computational system causes the computational system to receive a sequence of optical images. The optical images comprise optical imaging data of at least a part of a tissue of interest of the subject, which is of interest for the photoplethysmography signal extraction. Execution of the machine-executable instructions further causes the computational system, for the sequence of optical images, to divide a respective image region comprised by each optical image of the sequence into a plurality of subsets of pixels. Execution of the machine-executable instructions further causes the computational system to determine for at least some of the subsets dynamic characteristics of optical signals comprised by the respective subsets. Each of the dynamic characteristics is described by an intrinsic mode function. The respective intrinsic mode function is indicative of a temporal signal dynamic of the optical signal within the respective subset over the sequence of optical images.

Execution of the machine-executable instructions further causes the computational system to cluster the plurality of subsets, using the determined dynamic characteristics, to group the subsets of pixels into clusters with similar signal dynamics. Execution of the machine-executable instructions further causes the computational system to select a cluster from the resulting clusters comprising a cluster characteristic satisfying a pre-defined selection criterium. Execution of the machine-executable instructions further causes the computational system to identify the pixels comprised by the selected cluster as the region of interest of the optical images for extracting the photoplethysmography signal of the subject.

BRIEF DESCRIPTION OF THE DRAWINGS

In the following preferred embodiments of the invention will be described, by way of example only, and with reference to the drawings in which:

Fig. 1 shows a flow chart which illustrates an example of a method for determining a region of interest for extracting a photoplethysmography signal;
Fig. 2 shows a flow chart which illustrates an example of a method for determining and using a region of interest for extracting a photoplethysmography signal;
Fig. 3 shows a flow chart which illustrates an example of a method for determining a region of interest for extracting a photoplethysmography signal;
Fig. 4 shows a flow chart which illustrates an example of a method for determining intrinsic mode functions for subsets using an empirical mode decomposition;
Fig. 5 illustrates an exemplary computational system for determining a region of interest for extracting a photoplethysmography signal;
Fig. 6 illustrates an exemplary medical imaging system; and
Fig. 7 illustrates an exemplary medical imaging system in form of a magnetic resonance imaging system.

DETAILED DESCRIPTION OF EMBODIMENTS

Like numbered elements in these figures are either equivalent elements or perform the same function. Elements which have been discussed previously will not necessarily be discussed in later figures if the function is equivalent.

Examples provide a beneficial approach for determining a region of interest for extracting a photoplethysmography signal of a subject within optical images. These optical images are acquired using a remote optical sensor arranged relative to the subject at a position, which is spaced apart from the subject. A field of view of the optical sensor may cover at least a part of a tissue of interest of the subject, which is of interest for the photoplethysmography signal extraction. A sequence of optical images acquired by the optical sensor may be received for the determining of the region of interest. The optical images comprise optical imaging data of the at least part of the tissue of interest of the subject, which is of interest for the photoplethysmography signal extraction. The tissue of interest may, e.g., be skin tissue, mucous membranes, and other translucent tissue of the subject.

For the sequence of optical images, an image region comprised by each optical image of the sequence is divided into a plurality of subsets of pixels. From this plurality of subsets one or more subsets may be determined as a region of interest suitable for a PPG signal extraction and analysis using a detection of pulsatility within the optical signals comprised by the individual subsets over the images of the sequence.

For at least some of the subsets dynamic characteristics of optical signals comprised by the respective subsets are determined. Such dynamic characteristics may be determined for all the subsets or for a selection of subsets. Applying a pre-selection on the plurality of subsets, those subsets may be identified, which have high likelihood of corresponding to homogeneous areas of the tissue of interest comprising an optical signal representing a relevant pulsatility. Each of the dynamic characteristics is described by an intrinsic mode function determined using an empirical mode decomposition. For example, the determining of the dynamic characteristics for the at least some subsets in the sequence of optical images comprises determining per subset a spatial average of signal intensity of the optical signal over the pixels of the respective subset. A series over time, i.e., over the sequence of optical images, of the resulting mean signal intensities is used for the empirical mode decomposition for determining the intrinsic mode functions.

The respective intrinsic mode function is indicative of a temporal signal dynamic of the optical signal within the respective subset over the sequence of optical images. Using the empirical mode decomposition, a set of candidate intrinsic mode functions for describing temporal signal dynamic of the respective optical signal within the respective subset may be determined, e.g., iteratively. From this set of candidate intrinsic mode functions, an intrinsic mode function descriptive of a prominent component of the temporal signal dynamic may be selected and assigned to the respective subset.

The plurality of subsets may be clustered, using the determined dynamic characteristics, i.e., the intrinsic mode functions assigned to the subsets, to group the subsets of pixels into clusters with similar signal dynamics. Furthermore, additional characteristics, like energies and/or frequencies of the intrinsic mode functions and/or energies of optical signals detected by the subsets may be used for the clustering. One or more clusters may be selected from the resulting clusters comprising a cluster characteristic satisfying a pre-defined selection criterium. For example, the cluster characteristic comprises one or more of the following: cluster size, cluster morphology. For example, a largest cluster or are most similar to a predefined target shape may be selected from the plurality of clusters resulting from the clustering of the subsets. For example, for each cluster a shape measure representative of a shape of the image area formed by the pixels of the respective cluster may be determined. The selection of the cluster may than be based on the shape measure of the selected cluster being consistent with the predefined target shape of an area of the tissue of interest, e.g., such as an oval or ellipse shape indicative of the exposed skin of the face or of the forehead of the subject.

For example, the selecting of the cluster may comprise determining a ranking of the clusters of resulting from the clustering. This ranking may, e.g., combine a first score based on a size of the cluster and a second score based on the shape measure of the cluster. The ranking may, e.g., be used to determine the selection, e.g. selecting the highest or the top-N (e.g. 2, 3, ...) highest ranked clusters.

The pixels comprised by the selected one or more clusters are identified as the region of interest of the optical images for extracting the photoplethysmography signal of the subject.

Examples may have the beneficial effect that an obtaining of an accurate and/or stable PPG signal may be enabled, e.g. by reducing and/or avoiding carrying over nuisance signals, e.g. random noise or systematic errors, to a PPG output signal being inferred from the raw data stream of optical imaging data.

An output signal thus provided may, e.g., require less post-processing, e.g. further cleaning of the signal. This may improve the efficient use of computational resources, e.g. processing power, memory resources, power consumption and the like, and/or may improve the accuracy and stability of results derived from the output. For example, many signal cleaning strategies require that the measured disturbance signal and the disturbance in the raw PPG are synchronous, a condition which is not always satisfied. By careful selection of pixels from which the signal is derived, a more stable and representative PPG signal may be obtained, in which residual nuisance components may be generally relatively smaller and less random.

This determining of the region of interest may be executed by a computational system. The computational system may, e.g., be a standalone computational system or a computational system comprised by a medical imaging system. The medical imaging system may, e.g., further comprises a medical sensor system configured for acquiring medical imaging data of a subject and the optical sensor configured for acquiring the optical images of the subject. For example, a PPG signal and/or PPG markers identifying one or more stages in the cardiac cycle of the subject may be determined using the region of interest. These PPG signal and/or PPG markers may be used for controlling the medical sensor system, e.g., for triggering a medical image data acquisition using the medical sensor system.

Examples may have the beneficial effect of enabling a determining of a region of interest based on a selection of pixels representative of a substantial PPG pulsative signal. Particularly, a-priori knowledge may be used to prevent undesirable signal components, i.e. nuisance signals, noise and/or other disturbances, from contributing to the extracted PPG signal. For example, a refined pixel screening process may be applied to select a suitable region of interest, based, e.g., on morphological knowledge of the characteristics of promising candidate areas may be used for the region of interest determination. Prior knowledge of the properties of the pulsatility associated with PPG may also or alternatively be used.

Examples may provide for an improved region of interest detection using intrinsic mode functions determined using an empirical mode decomposition. This approach may be particularly beneficial in cases of arrythmias. Such an improved region of interest may, e.g., be used to facilitate a triggering of a medical imaging data acquisition using the PPG signal, e.g., in case MRI, CT, PET and/or SPECT data acquisition. Moreover, examples may provide for a computationally low-cost implementation of the improved region of interest detection using intrinsic mode functions based on insights in processing mechanisms and application areas of the detection.

Examples may have the beneficial effect that a detection of pulsatility and the region of interest detection based thereon may remain undisturbed by irregular beats. In addition, examples may satisfy the additional requirement of a relatively low computational demand.

An empirical mode decomposition (EMD), e.g., unlike a Fourier analysis, does not require a strict periodicity. EMD is not sensitive to shortening or lengthening of one or more cardiac cycles. A pulsatility detection based on intrinsic mode may be implemented in form of a decomposition pattern selection task. Taking into account the nature of the EMD decomposition and/or the intended application, i.e., describing dynamic characteristics of a PPG signal representing a heartbeat, a low-cost implementation may be configured. For this purpose, e.g., measures for limiting the number of intrinsic mode functions being generated using the empirical mode decomposition and consequently the number of iteration loops of the empirical mode decomposition being executed. A first measure may, e.g., comprise creating an appropriate input signal. A second measure may, e.g., comprise a truncation of the empirical mode decomposition as soon as all decomposition patterns, i.e., intrinsic mode functions, relevant for the pulsatility detection have been obtained. An induced effect of the limiting the intrinsic mode functions, may be that the process of selecting of an appropriate intrinsic mode function from the set of candidate intrinsic mode functions determined using the empirical mode decomposition may become computationally less demanding, e.g., since there may be less comparisons to be evaluated, and/or more robust, e.g., since less confusions may be possible, compared to an evaluation of an unlimited set of candidate the intrinsic mode functions.

An acquisition of a photoplethysmography (PPG) signal in form of an optical signal acquired using a remote optical sensor, which is arranged spaced apart from a person, of which the signal is to be acquired, is referred to as remote-PPG, also called camera-PPG or video-PPG. Such remote-PPG provides for a method of measuring in a non-contact way the PPG signal of a person. The information contained in the PPG signal may be used in various ways, one of these being a triggering or gating of a medical image acquisition, like a magnetic resonance image (MRI) data acquisition.

Remote-PPG may have several advantages over conventional contact-based PPG, comprising, e.g., an ability to capture a larger area of a tissue of interest, which may, e.g., be helpful in cases, where the tissue of interest is not evenly illuminated, and/or an ability to capture multiple PPG signals from different parts of the tissue of interest simultaneously, e.g., to improve an accuracy of the result by averaging.

For this purpose, first a region of interest may have to be determined in the acquired optical images. These images are acquired in form of a sequence of images, e.g., a video, in order to be able to determine temporal signal dynamics of the PPG signal. This region of interest is a region of the images comprising optical imaging data of tissue of interest for extracting a PPG signal, e.g., skin tissue from which the PPG signal may be extracted. After having identified the region of interest in a sequence of images, a PPG signal provided by the optical signal data comprised by the pixels of the identified region of interest may be determined. This determining may, in case of a polychromatic optical signal, e.g., comprise a downmixing of the polychromatic signal and/or, e.g., a post-processing of the signal to clean the signal from undesired/disturbing components. From the resulting PPG signal, features of the PPG signal may be extracted. For example, a pulse rate may be extracted. In case the PPG signal is to be used for triggering a medical imaging data acquisition, e.g., an MRI data acquisition, PPG markers identifying one or more stages in a cardiac cycle of a subject may be extracted. These markers may then be fed to a trigger module that it configured to, e.g., trigger an MRI pre-pulse and to control data acquisition windows of the MRI data acquisition. Such marker may, e.g., comprise a position of a maximum of the PPG signal, a negative-going zero-crossing of the PPG signal, and/or a positive-going zero-crossing of the PPG signal.

For an MRI data acquisition, the determining of the region of interest may, e.g., rely partly on knowledge of the MRI scanning environment, which may be typically fixed. Large parts of the optical images may show section of the technical components of the MRI system rather than tissue of interest of a subject. These sections depicting components of the MRI system may be discarded upfront. Furthermore, level information may be used. This level information may, e.g., define required and/or expected level of illumination. The region of interest should, e.g., be region of the images not too dark and/or not too bright in case of well-illuminated tissue of interest, like skin. Furthermore, pulsatility information may be used in case of tissue in form of living skin. Since a PPG signal may be small in general compared to an environmental light, a precise control over the illumination source used for the acquisition of the optical images may be beneficial for enabling a continuous, accurate monitoring. The dynamic range of the camera-based PPG signal itself may be rather small, i.e. in the order of $10^{-3}$ relative to the mean illumination (mean pixel value offsets) and also small to other factors that could influence the detected pixel signals.

For example, a monochromatic infrared (IR) illumination source may be used. In this case, the optical images may be IR images comprising IR data. For example, a single channel near-infrared (NIR) illumination source and an optical sensor configured for acquiring NIR optical imaging data.

The optical sensor may be an infrared sensor, e.g., operating in the near infrared range, e.g. in a range of 800 to 850 nm. Using an infrared sensor, a PPG signal may be acquired without requiring an adjustment of the visible lighting conditions, and/or without being influenced by ambient light in the visible spectrum. Since the human eye is not sensitive to infrared light, the subject being monitored is therefore not affected by unpleasant, disturbing and/or uncomfortably bright light. However, examples are not limited thereto. For example, it is also possible to extract a PPG signal from imaging in the visible range. For example, the light absorption of (de) oxygenated hemoglobin is stronger in the (near) infrared wavelength range than for visible light, e.g., red light, and infrared light may penetrate deeper into skin, since, e.g., the influence of melanin in the skin may be less pronounced. Also, infrared sensors, e.g. based on semiconductor pixel detectors, may have a higher sensitivity in the NIR range than for wavelengths above 900 nm, which may result in a higher signal to noise ratio and/or better operation when the tissue of interest is not ideally illuminated. Light ranges other than IR or NIR may also be used for PPG signal detection, e.g., the red or green wavelength range.

The optical signals of the different pixels of the region of interest may be combined to construct the PPG signal. Disturbing signal components may be eliminated from the signal by signal-decomposition techniques. Such disturbances may, e.g., come from motion. Therefore, motion signals in the images may be measured and eliminated from the raw optical signal. Similarly, light variations in non-skin areas may be measured and eliminated from the optical signal.

In case of living tissue, like living skin, the optical image data within the region of interest for the photoplethysmography signal extraction may be expected to comprise a temporal signal dynamic representing the pulsatility. This pulsatility may beneficially be described by one or more intrinsic mode functions. Using intrinsic mode functions may have the beneficial effect that even irregular heart rhythms, i.e., arrythmias, may be precisely decried. For example, cardiac examinations with MRI, CT, PET and/or SPECT systems need to be operable in case of patients with arrythmias.

Using intrinsic mode functions determined using an empirical mode decomposition may, e.g., over a frequency analysis based on a Fourier analysis. For example, using intrinsic mode functions may have the beneficial effect that due to an improved region of interest determination with more subsets being may result in a lager region of interest with a higher signal-to-noise ratio in the detected PPG signal. This in turn may result in an enhanced performance of information derived from the PPG signal. Considering the full processing and data acquisition chain, this may, e.g., lead to a higher medical image quality and/or to a shortening of the scanning process, which are both beneficial effects.

Thus, problems in handling arrythmias, like, e.g., in case of a Fourier analysis, may be avoided. When an arrythmia occurs, the Fourier analysis, e.g., may have a problem of wrong subsets being chosen, when determining a region of interest, or even no subsets being chosen at all. This error may last for some time as the Fourier analysis needs a sufficient observation time to be accurate and stabilization mechanisms may be used to prevent random on- and offset in chosen subsets. This may have the consequence, that in case of a Fourier analysis, once an incorrect discarding of a subset for the determination of the region of interest occurred, e.g., due to non-detected pulsatile within the respective subset, the subset may remain unused for a relatively long period of time. However, fewer selected subsets may result in a smaller region of interest with a smaller signal-to-noise ratio in the detected PPG signal and in turn may result in a degraded quality of information derived from the PPG signal. Considering the full processing and data acquisition chain, this may lead to either a poor medical image quality and/or to a lengthening of the scanning process, which are both undesired effects.

An empirical mode decomposition (EMD) is a method, which allows for breaking down a signal without leaving the time domain. Using the EMD method, any complicated data set can be decomposed into a finite and often small number of components. The EMD filters out functions, which are referred to as intrinsic mode functions that form a complete and nearly orthogonal basis for the original signal. Completeness may be based on the method of the EMD, since the way a signal is decomposed may imply completeness. The resulting intrinsic mode functions are therefore sufficient to describe the original signal, even though they are not necessarily orthogonal. The fact that the intrinsic mode functions into which the signal is decomposed are all in the time-domain and of the same length as the original signal allows for varying frequency in time to be preserved. With the decomposition being based on the local characteristic time scale of the signal, it may even be applied to nonlinear and nonstationary processes.

An intrinsic mode function (IMF) is defined as a function that satisfies the following requirements: In the whole data set, the number of extrema and the number of zero-crossings must either be equal or differ at most by one. Furthermore, at any point, the mean value of the envelope defined by the local maxima and the envelope defined by the local minima is zero. By definition, an intrinsic mode function is any function with the same number of extrema and zero crossings, whose envelopes are symmetric with respect to zero.

An intrinsic mode function represents a simple oscillatory mode, like, e.g., a simple harmonic function, but it is much more general, since amplitude and frequency of an intrinsic mode function may vary with time.

The procedure of extracting an intrinsic mode function is referred to as sifting. The sifting comprises the following: All local extrema in a given data set are identified, all local maxima are connected, e.g., using a cubic spline line, as an upper envelope, and all local minima are connected, e.g., using a cubic spline line, as a lower envelope. The upper and lower envelopes should cover all the data between them. Their mean may be denoted by $m_1$. The difference between the data $X(t)$ and $m_1$ is a first component hi, i.e., $X(t) - m_1 = h_1$. Ideally, $h_1$ should already satisfy the definition of an intrinsic mode function, since the construction of $h_1$ described above should have made it symmetric and having all maxima positive and all minima negative. In the subsequent sifting process, $h_1$ may be treated as a proto-intrinsic mode function. In the next step, $h_1$ is treated as data $h_{11}$, i.e., $h_1 - m_{11} = h_{11}$. After repeated sifting up to k times, $h_{1(k-1)}$ becomes an intrinsic mode function, i.e., $h_{1(k-1)} - m_{1k} = h_{1k}$. Then, $h_{1k}$ may be designated as the first intrinsic mode function of the data, i.e., $c_1 = h_{1k}$. Thus, the first intrinsic mode function carries the most oscillating, i.e., high-frequency, components of a signal being decomposed. The first intrinsic mode function is separated from the rest r1 of the data, i.e., $X(t) - c_1 = r_1$, for which the procedure of determining an intrinsic mode function is repeated. Thus, the determining of intrinsic mode is iteratively repeated for $r_j$, i.e., $r_1 - c_2$

= $r_2$, ..., $r_{n-1}$ - $c_n$ = $r_n$. The result is a set of intrinsic mode function, the number of which depends on the original signal.

Stoppage criteria determining the number of sifting steps to produce an intrinsic mode function, may e.g., comprise one of the following: standard deviation, S number criterion, threshold method, energy difference tracking.

For example, the region of interest may be determined for usage for extracting a photoplethysmography signal in order to determine features of the subject's cardiac cycles.

In an example, the determining of the intrinsic mode functions further comprises a pre-processing of the optical signals comprised by the plurality of subsets. The pre-processing comprises removing signal components of the optical signals with frequencies above an upper limit of a predefined band of heart rates of interest. Example may have the beneficial effect that, by removing such signal components of the optical signals with frequencies above an upper limit of a predefined band of heart rates of interest, the determining of the intrinsic mode functions may be accelerated. Signal components with frequencies above an upper limit of a predefined band of heart rates of interest may not be suitable for determine features of the subject's cardiac cycles.

For example, the predefined band of heart rates of interest is a band from 5 beats per minute to 300 beats per minute. For example, the predefined band of heart rates of interest is a band from 30 beats per minute to 150 beats per minute. For example, the predefined band of heart rates of interest is a band from 50 beats per minute to 100 beats per minute.

In an example, a selecting of the at least some subsets comprises per subset of the plurality of subsets a determining of a ratio of a pre-processed energy of the pre-processed optical signal of the respective subset to an initial energy of the optical signal before the pre-processing. The selected subsets from the plurality of subsets are subsets for which the ratio reaches a first predefined threshold. Examples may have the beneficial effect that for the determining of the intrinsic mode functions only such subsets may be taken into account, for which the ratio of the pre-processed energy to the initial energy of the optical signal reaches first predefined threshold. Only subsets may be selected for which the ratio is sufficiently large, i.e., for which the pre-processed optical signal to be used for the empirical mode decomposition is large enough compared to the total initial optical. For example, it may be required that the pre-processed optical signal is significantly larger than the signal components removed by the pre-processing, which may be considered as noise.

In an example, wherein the empirical mode decomposition for determining the intrinsic mode functions comprises for each of the subsets an iteratively determining of one or more candidate intrinsic mode functions, from which the intrinsic mode function is selected. The iterative determining of the candidate intrinsic mode functions may, e.g., start with determining a candidate intrinsic mode function comprises a largest frequency and then successively continue determining candidate intrinsic mode function with decreasing frequencies.

In an example, from the one or more candidate intrinsic mode functions the candidate intrinsic mode function with a largest energy is selected as the intrinsic mode function. Examples may have the beneficial effect that the candidate intrinsic mode function with a largest energy may be a prominent component of the optical signal, i.e., the PPG signal, being decomposed. Such a prominent component may be a suitable for describing a prominent dynamic characteristic of the optical signal within the respective subset.

In an example, the determining of the candidate intrinsic mode functions comprises per candidate intrinsic mode function determining a ratio of energy of the respective candidate intrinsic mode function to a pre-processed energy of the pre-processed optical signal of the subset, for which the respective candidate intrinsic mode function is determined. Candidate intrinsic mode functions, for which the ratio reaches a predefined second threshold, are discarded for the selection of the intrinsic mode function. Example may have the beneficial effect that only candidate intrinsic mode, which are a suitable large component of the pre-processed optical signal may be taken into account for the selection of the intrinsic mode function to be used as a description of a prominent dynamic characteristic of the subset and to be assigned to the respective subset.

In an example, the determining of the candidate intrinsic mode functions further comprises determining frequencies of the candidate intrinsic mode functions. For example, candidate intrinsic mode functions with frequencies above the upper limit of the predefined band of heart rates of interest are discarded for the selection of the intrinsic mode function. Example may have the beneficial effect that candidate intrinsic mode functions with frequencies, which are unable to describe a cardiac cycle with a frequency with the predefined band of heart rates of interest, are discarded. For example, a first iterative step of the empirical mode decomposition may result in such a candidate intrinsic mode function with a frequency above an upper limit of the predefined band of heart rates of interest.

For example, candidate intrinsic mode functions with frequencies below a lower limit of the predefined band of heart rates of interest are discarded for the selection of the intrinsic mode function. Example may have the beneficial effect that candidate intrinsic mode functions with frequencies, which are unable to describe a cardiac cycle with a frequency with the predefined band of heart rates of interest, are discarded. For example, a last iterative step of the empirical mode decomposition may result in such a candidate intrinsic mode function with a frequency below a lower limit of the predefined band of heart rates of interest.

In an example, the iterative determining of candidate intrinsic mode functions is terminated upon reaching a termination criterium.

For example, the termination criterium is a determining of an intrinsic mode function with a frequency below the lower limit of the predefined band of heart rates of interest. Since the frequency of the intrinsic mode functions is decreasing from iterative step to iterative step, further intrinsic mode function will have even lower frequencies. Example may have the beneficial effect that a determining of candidate intrinsic mode functions with frequencies, which are unable to describe a cardiac cycle with a frequency with the predefined band of heart rates of interest, may be avoided by terminating the iterative determining of candidate intrinsic mode functions.

For example, the termination criterium is a determining of an intrinsic mode function with a frequency within a predefined frequency band above the lower limit of the predefined band of heart rates of interest. Example may have the beneficial effect that a determining of a candidate intrinsic mode function with a frequency below the predefined band of heart rates of interest may be avoided by termination the iteration before the frequencies of the candidate intrinsic mode functions drop below the lower limit of the predefined band of heart rates of interest. The predefined frequency band above the lower limit of the predefined band of heart rates of interest may, e.g., be chosen such that for a candidate intrinsic mode function with a frequency within this band above the lower limit next candidate intrinsic mode function may most likely have a frequency below the lower limit.

For example, the termination criterium is a sum of energies of the candidate intrinsic mode functions iteratively determined reaching a predefined difference to the pre-processed energy of the pre-processed optical signal of the respective subset. Example may have the beneficial effect that a determining of a candidate intrinsic mode function with a frequency below the predefined band of heart rates of interest may be avoided by termination the iteration before the frequencies of the candidate intrinsic mode functions drop below the lower limit of the predefined band of heart rates of interest. The sum of energies of the candidate intrinsic mode functions iteratively determined reaching the predefined difference to the pre-processed energy may have the effect that the remaining signal components of pre-processed signal, for which no candidate intrinsic mode functions have been determined yet, may comprise an insufficient amount of energy for determining any candidate intrinsic mode function with a significant level of energy compared to the candidate intrinsic mode functions already determined. Thus, any further candidate intrinsic mode function determinable may have an energy too low to be selected as the intrinsic mode function for describing the dynamic characteristic of the respective optical signal. Therefore, the iteration may be terminated before dynamic characteristics of optical signals are determined, which have a high likelihood of being irrelevant for the further analysis.

In an example, the execution of the machine executable instructions further causes the computational system to discard from the determining of the intrinsic mode functions such subsets, which satisfy one or more predefined signal criteria for one or more signal characteristics of the optical signals. The signal characteristics are determined on a subset-per-subset basis. The one or more signal criteria for example comprise a mean signal intensity per subset. The one or more signal criteria for example comprise a spread of signal intensity per subset. A spread may, e.g., be determined using a standard deviation. The one or more signal criteria for example comprise at least one value indicative of a motion associated with the respective subset. Such a value indicative of a motion may, e.g., be motion vector, a component of a motion vector or an absolute value of a motion vector. A motion vector may be determined using an optical flow algorithm.

Example may have the beneficial effect that subsets may be discarded, which have a low likelihood of corresponding to homogeneous areas of the tissue of interest. It may be expected that subsets of the region of interest comprise a relevant signal intensity resulting from a pulsatility the tissue of interest. In case the signal intensity is too small, the subset may be expected to not depicting a part of the tissue of interest. For example, the signal intensity condition, e.g., an averaged pixel signal intensity condition, may ensure lighting conditions that fall within a comfortable margin in a dynamic range of the optical sensor and that are consistent with typical characteristics of the tissue of interest under such lighting conditions.

If the spread of signal intensity of a subset is too large, e.g., reaches a predefined threshold, the subset may not represent a homogeneous area, for which a reliable analysis of the optical signal comprised by the respective subset may be possible. This spread may, e.g., be an indication of a high level of noise within the respective subset. Furthermore, a motion of the subset may spoil a reliable analysis of the optical signal comprised by the respective subset. Such a motion may, e.g., cause a high level of noise within the respective subset. When the spread and motion are below (respective) predetermined thresholds some level of stability for determining the optical signal may be assumed, e.g., the respective subset may not be excessively affected by various sources of noise and/or errors, nor by strong motion.

The subject's cardiovascular function described by the PPG signal may be used to monitor the subject's state, e.g. health, in a procedure and be taken into account in processing images obtained in a medical imaging procedure and/or to guide or control a medical imaging data acquisition.

In an example, the execution of the machine executable instructions further causes the computational system to extract the photoplethysmography signal from the region of interest identified within the optical images. The photoplethysmography signal being extracted is provided by the optical signal data comprised by the pixels of the identified region of interest. This exctraction may, in case of a polychromatic optical signal, e.g., comprise a downmixing of the polychromatic signal and/or, e.g., a post-processing of the signal to clean the signal from undesired/disturbing components. From the resulting PPG signal, e.g., features of the PPG signal may be extracted. For example, a pulse rate may be extracted. In case the PPG signal is to be used for triggering an MRI data acquisition, PPG markers identifying one or more stages in a cardiac cycle of the subject may be extracted. Such markers are points in time that identify one or more specific stages in the cardiac cycle. The markers may then be fed to a trigger module that it configured to, e.g., trigger an MRI pre-pulse and to control data acquisition windows of the MRI data acquisition. Such marker may, e.g., comprise a position, i.e. point in time, of a maximum of the PPG signal, a negative-going zero-crossing of the PPG signal, and/or a positive-going zero-crossing of the PPG signal.

Once a region of interest has been determined, there may be multiple options for extracting a PPG signal from this region of interest. For example, an average value of the optical signal acquired by the pixels selected as the region of interest may be determined and tracked over time, thereby creating the PPG signal. Since averages per subset for different points in time, i.e., for different optical images of the sequence of optical images, may already have been determined, when determining the region of interest, the average values of the subsets selected as the region of interest may be averaged per optical images, thus resulting in the PPG signal. Alternatively, the pre-processed signals determined per subset, when determining the region of interest, may be averaged for the subsets selected as the region of interest, in order to obtain a cleaner PPG signal, in which, e.g., high-frequency components are suppressed and/or filtered out. Instead of averaging, e.g., also a weighted averaging or nonlinear combinations of the aforementioned signals per subset or of the underlying pixels of the respective subsets may be used for determining the PPG signal. Respective weights may, e.g., be derived by re-using features of the selection mechanism used for selecting the subsets of pixels, which form the region of interest.

Optical images of the subject may be acquired, e.g., during a medical and/or diagnostic examination or intervention. The examination may, e.g., be a magnetic resonance imaging (MRI), computed tomography (CT), positron emission tomography (PET) or single-photon emission computed tomography (SPECT) data acquisition. Examples of interventions may, e.g., include therapeutic and related procedures, such as surgery and radiotherapy. Examples may also be used in general-purpose subject or patient monitoring, such as in an intensive care unit, a patient ward, or in a non-medical setting, e.g. in a nursing home. The PPG signal determined using the region of interest may, e.g., be used to monitor the health and/or state of the subject, and/or to control a system or device used for an examination or invention. The tissue of interest may be comprised by one or more body parts of interest of the subject. Such body parts may, e.g., comprise a face or a part thereof, e.g. a forehead, but are not necessarily limited thereto. For example, also the tissue, like skin, of another body part may be used, e.g., of an arm, a hand, a chest, a leg, a foot, an abdomen, etc.

In an example, wherein the execution of the machine executable instructions further causes the computational system to use the extracted photoplethysmography signal for controlling an acquiring of medical imaging data of the subject using a medical imaging system. The medical imaging data acquisition may, e.g., comprise an MRI data acquisition, a CT data acquisition, a PET data acquisition and/or a SPECT data acquisition.

For example, further sequences of further optical images may be received. In the further optical images, the determined region of interest may be identified and an intrinsic mode function descriptive of a dynamic characteristic an optical signal comprised by the determined region of interest within further images of a further sequence may be determined using an empirical mode decomposition. The resulting intrinsic mode function may, e.g., be used for controlling, e.g., triggering, a medical imaging data acquisition. The medical imaging data acquisition may, e.g., comprise an MRI data acquisition, a CT data acquisition, a PET data acquisition and/or a SPECT data acquisition.

Examples may have the have the beneficial effect that a remote imaging may be used for acquiring the optical imaging data, e.g., such that direct contact with the subject is not necessary and/or such that equipment may be placed at a substantial distance spaced apart from the subject. For example, in a medical environment, it may be preferable to keep the direct vicinity of the patient clear of obstructions, e.g. such that a procedure can be performed without unnecessary hindrance.

Furthermore, in medical imaging, the diagnostic imaging technology may, e.g., interfere with other electronics. In magnetic resonance imaging e.g., the hardware of the MRI sensor system, e.g. radiofrequency antennae and/or magnetic field gradient coils may interfere with a correct operation of further electronic devices. For example, the PPG optical sensor may pick up noise due to radiofrequency signals and/or be affected by magnetic fields, field gradients and/or dynamic field changes. Similarly, illumination sources used to illuminate the tissue of interest of the subject for PPG detection may be disturbed by the MRI hardware giving, e.g., rise to illumination changes that may disturb PPG detection.

Example may, e.g., enable an automatic, e.g., algorithmic, determining of a PPG signal from an observation of a subject using an optical sensor. In many environments, a suitable optical sensor may, e.g., already be present, or may be installed in an upgrade, such that embodiments may be applied to an existing system without complex and/or costly interventions.

Examples may have the beneficial effect that a signal may be determined from an observation using an optical sensor. The signal may be for triggering and/or controlling a system in a diagnostic imaging procedure using a scanner system, such as an MRI, CT, PET, or SPECT scanner, which may improve the diagnostic image quality. Such trigger signals may also be used in, e.g., radiotherapy and similar, e.g. therapeutic, procedures. Furthermore, such trigger signals may, e.g. not necessarily strictly limited to medical imaging applications. Furthermore, other areas of application for PPG acquisition are not necessarily excluded either.

For example, a determining of the image region being divided into the plurality of subsets and/or the dividing of the image region may comprise identifying anatomical key points in the sequence of optical images. The plurality of subsets may, e.g., be determined using the identified anatomical key points.

Anatomical key points are locations referenced to a portion of a subject's body. They may for example indicate the location of joints, location on the face, or other body portions. There currently exists commercial software which is able to automatically identify anatomical key points. For even video game systems such as the Microsoft Xbox have had such software capability built in. Neural networks, such as a convolution neural network trained using images labeled with anatomical key points may also be used.

For example, the determining of the image region and/or of the plurality of subsets may comprise fitting templates to the identified anatomical key points in the sequence of optical images. Templates may, e.g., be fit and/or deformed to the anatomical key points to provide the region of image and/or the plurality of subsets. This may provide for an effective means of identifying regions of the subject to use for the plurality of subsets.

For example, a neural network configured to output the anatomical key points in response to receiving the sequence of optical images as input may be used. A neural network trained with images labeled with the anatomical key points may be used. Various types of neural networks may be used. For example, a convolutional neural network, a U-Net, a ResNet, or a network with fully connected layers may be configured to receive an image as input and to output the location of the anatomical key points.

For each the subset of the plurality of subsets an average property of the respective subset such as the brightness, contrast, location in the color spectrum, change in color, or other property may be recorded as a function of time using multiple optical images. This sequence of values may then be used as the optical signal being analyzed using an empirical mode decomposition, in order to determine th region of interest.

For example, the dividing of the image region may into the plurality of subsets may comprise forming a partition of the respective image region into blocks or patches based on a spatial distance metric and/or into subsets based on an image codomain distance and/or based on a combination thereof, such that each subset groups pixels, which are close together in space and/or in value. For example, different subset sizes may be considered, e.g., in an optimization strategy, the grouping of pixels into the subsets may switch, or shift in weight, from forming subsets that are more or only based on distance in the spatial domain to forming subsets that are more or only based on distance in the image codomain, e.g., the pixel value domain.

For example, the dividing of the image region may into the plurality of subsets may comprise an image subtraction between an image acquired with the subject present and a blank image acquired without the subject present, and/or a similar image background compensation technique, so as to detect the respective image region to be divided.

For example, the step of dividing the image region into the plurality of subsets may be executed repeatedly, e.g., to apply an optimization strategy, such that different iterations correspond to different values of at least one optimization parameter representative of at least the size of the subsets. The dividing may further comprise evaluating a quality metric based on the subsets obtained in each iteration so as to select the parameter value for which a sufficient or optimal value of the quality metric is obtained, and using the plurality of subsets obtained for said selected parameter value in the further steps of the determining of the region of interest, e.g., such that the clustering, and therefore also the region of interest determined therefrom, is based on subsets corresponding to said parameter choice.

For example, the at least one optimization parameter may further comprise a size of a temporal observation window used for determining the at least one dynamic characteristic value per subset. For example, the quality metric used in said optimization strategy may comprise or consist of a signal-to-noise ratio.

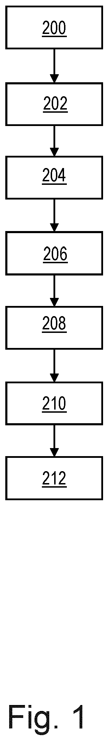

Fig. 1 illustrates an example of a method for determining a region of interest for extracting a photoplethysmography signal of a subject within optical images. The method may, e.g., executed by a computational system executing machine executable instructions defining the method. The computational system may, e.g., be comprised by a medical imaging system, which may further comprise a medical sensor system configured for acquiring medical imaging data of the subject and/or an optical sensor configured for acquiring optical images of the subject.

In block 200, a sequence of optical images is received. The optical images comprise optical imaging data of at least a part of a tissue of interest of the subject, which is of interest for the photoplethysmography signal extraction. In block 202, for the sequence of optical images, a respective image region comprised by each optical image of the sequence is divided into a plurality of subsets of pixels. In block 202, for example a pruning of subsets may be executed. Thus, subsets that have a low likelihood of corresponding to homogeneous areas of the tissue of interest may be excluded from further consideration, when determining the region of interest. For this purpose, subsets may be discarded, which satisfy one or more predefined signal criteria for one or more signal characteristics of the optical signals. The signal characteristics are determined on a subset-per-subset basis with the one or more signal criteria comprising one or more of the following: a mean signal intensity per subset, a spread of signal intensity per subset, and at least one value indicative of a motion associated with the respective subset. In block 206, for at least some of the subsets dynamic characteristics of optical signals comprised by the respective subsets are determined. Each of the dynamic characteristics is described by an intrinsic mode function. The respective intrinsic mode function is indicative of a temporal signal dynamic of the optical signal within the respective subset over the sequence of optical images.

In block 208, the plurality of subsets, using the determined dynamic characteristics, is clustered to group the subsets of pixels into clusters with similar signal dynamics. In block 208, a cluster from the resulting clusters comprising a cluster characteristic satisfying a pre-defined criterium is selected. In block 212, the pixels comprised by the selected cluster are identified as the region of interest of the optical images for extracting the photoplethysmography signal of the subject.

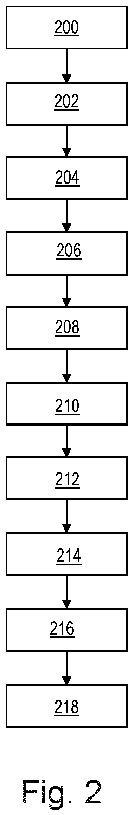

Fig. 2 illustrates an example of a method for determining and using a region of interest for extracting a photoplethysmography signal of a subject within optical images. The method may, e.g., executed by a computational system executing machine executable instructions defining the method. The computational system may, e.g., be comprised by a medical imaging system, which may further comprise a medical sensor system configured for acquiring medical imaging data of the subject and/or an optical sensor configured for acquiring optical images of the subject.

The determining of the region of interest for extracting the photoplethysmography signal of Fig.2 according to blocks 200 to 212 corresponds to the determining of the region of interest according to block 200 to 212 of Fig. 1. The resulting region of interest may then be used to extract the photoplethysmography signal from the region of interest identified within the optical images. In block 214, further sequences of optical images may be received. Alternatively or alternatively, the photoplethysmography signal may be extracted from the sequence of optical images received in block 200, which has been used to determine the region of interest. In block 216, the region of interest determined before in block 212 is identified in the further sequences of optical images.

From the pixels comprised by the region of interest, a photoplethysmography signal is created as a series over time, i.e., over the optical images of the further sequences. The optical images may be provided as video frames of a video stream. For example, the photoplethysmography signal may be provided by a mean value of signal intensities of the pixels averaged over the region of interest at each optical image of a sequence of optical images or by a processed version or by a filtered version thereof. In block 216, the extracted photoplethysmography signal may be used, e.g., for controlling an acquiring of medical imaging data of the subject using the medical imaging system.

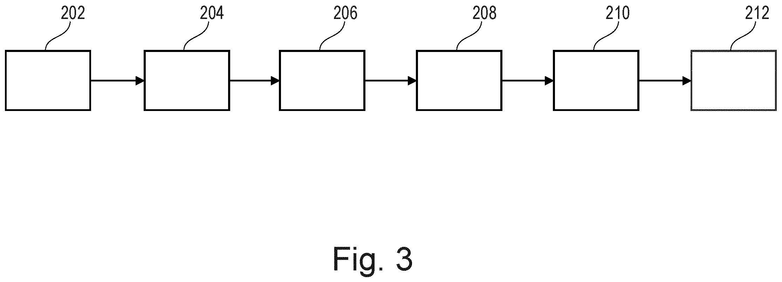

Fig. 3 illustrates a further example of a method for determining a region of interest for extracting a photoplethysmography signal of a subject within optical images. The method for determining the region of interest according to Fig. 3 corresponds to the method of Fig. 1.

In block 202, images of a received sequence of images or a part of each of these images, i.e., an image region, is divided into a plurality of subsets of pixels. These subsets may also be referred to as patches. The image region, if not corresponding to the complete image, may be determined using segmentation. In block 204, the subsets are pruned. For this purpose, e.g., a mean signal intensity per subset and/or a spread of signal intensity per subset may be determined. Furthermore, e.g., a value, e.g., a metric, indicative of a motion associated with the respective subset may be determined. If an optical signal comprised by a subset, i.e., signal characteristics of the respective optical signal, satisfies certain criteria, the subset under consideration may be considered as a homogeneous non-moving subset. Such homogeneous non-moving subsets may be used for further analysis, while subsets not satisfying the criteria may be discarded from further analysis. For the later, a likelihood of corresponding to a homogeneous area of the tissue of interest may be low.

The criteria for assessing the subsets may, e.g., comprise one or more of the following criteria: The mean signal intensity may be required to be sufficiently large; the spread may be required to be sufficiently small; and values indicative of a motion of the subset, i.e., motion indicators, may be required to be sufficiently small.

For example, a spatial average of signal intensity of the optical signal over the pixels per subset and image may be determined. The sequence of images then provides a temporal development of the spatial average of signal intensity of the optical signal within the respective subset.

In block 206, intrinsic mode functions are be determined for the remaining, i.e., non-discarded subsets. These intrinsic mode functions are descriptive of dynamic characteristics of optical signals comprised by the respective subsets and determined using an empirical mode decomposition. If a sufficiently distinct intrinsic mode functions is determined, the subset may be associated with the frequency of the respective intrinsic mode function. For example, a plurality of candidate intrinsic mode functions, e.g., with frequencies within a predefined band of heart rates of interest. From these candidate intrinsic mode functions, the respective intrinsic mode function associated with the subset may be determined. For example, an intrinsic mode function with a largest energy may be selected. In addition, it may be required that the energy of the selected intrinsic mode function relative to the energy of the rest of the candidate intrinsic mode function and/or relative to a candidate intrinsic mode function second in energy reaches a predefined threshold, i.e., is sufficiently large. The frequency of the intrinsic mode function selected for the respective subset may also be referred to as the subset or patch frequency. In block 208, these subset frequencies and thus the subsets may be clustered. In block 210 a cluster may be selected, e.g., a on its size and/or its morphology. For example, a largest cluster may be selected from the clusters resulting from block 208. The pixels and/or subsets belonging to the selected may be identified as the region of interest (RoI) in block 212. Using intrinsic mode functions resulting from an empirical mode decomposition may have the beneficial that the results may not be disturbed by an occurrence of a shortening or lengthening of a single cardiac cycle. For example, the prominence of individual intrinsic mode functions in the predefined band of heart rates may not be disturbed by such variations of the length of cardiac cycles.

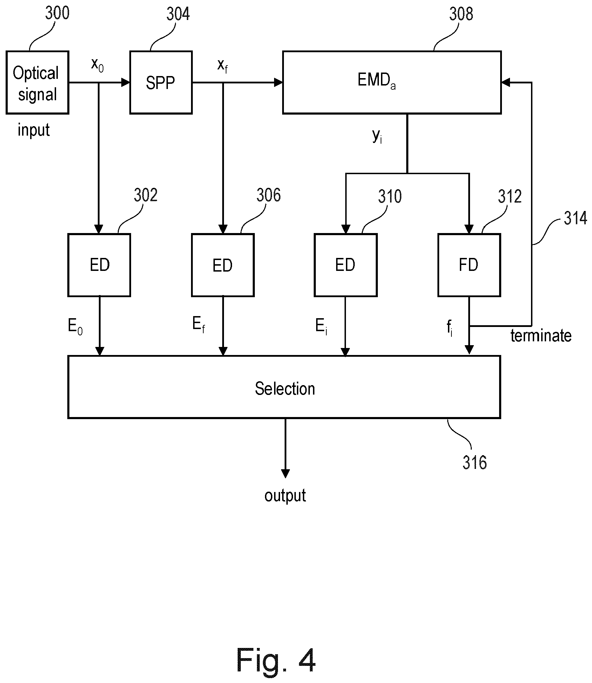

Fig. 4 illustrates a method for determining intrinsic mode functions for subsets using an empirical mode decomposition (EMD). In Fig. 4 an exemplary empirical mode decomposition mechanism including decomposition pattern selection criteria is used for determining the intrinsic mode functions. The sequence of optical images provides data representing a temporal dynamic of the optical signals within the subsets. The intrinsic mode functions determined for the individual subsets using the empirical mode decomposition are indicative of this temporal signal dynamic of the optical signals within these individual subsets over the sequence of optical images.

As an input an optical signal $x_0$ of a subset, i.e., a photoplethysmography (PPG) segment, may be provided in block 300. This signal, may, e.g., be provided in form of values of a spatial average of signal intensity of the optical signal over the pixels of the respective subset for different points in time. The data for the different points in time may be provided by the different images of the sequence of images. For each of these images a spatial average of signal intensity of the optical signal over the pixels of the respective subset may be provided resulting in a representation of a temporal development of the optical signal within the respective subset.

This initial signal $x_0$ may be input into an energy determining module ED in block 302 configured for determining an initial energy $E_0$ of the initial signal $x_0$. Furthermore, the initial signal $x_0$ may also be input into a signal pre-processing module SPP in block 304 configured for a pre-processing of the initial signal $x_0$. The SPP module may comprise a filter module configured for removing signal components of the initial signal $x_0$ above an upper limit of the predefined band of heart rates of interest. The filter module may, e.g., be implemented in form of a low-pass filter tuned at a cut-off frequency at or slightly above a highest heart rate of interest of the predefined band of heart rates of interest, e.g., 100 bpm. The SPP module may optionally also be configured to include a removal of frequency components of the initial signal $x_0$ below a lower limit of the predefined band of heart rates of interest, i.e., a lower limit of the predefined band of heart rates of interest. As an output of the SPP module a pre-processed signal $x_\varepsilon$ may be provided.

This pre-processed signal $x_f$ may be input into an energy determining module ED in block 306 configured for determining a pre-processed energy $E_\varepsilon$ of the pre-processed signal $x_\varepsilon$. The pre-processed signal $x_\varepsilon$ may further be input into an EMD analyzer module ($EMD_a$) in block 308. The EMD analyzer module may be configured to execute an empirical mode decomposition, which is implemented as an iterative process. As a result, the EMD analyzer module may output iteratively intrinsic mode functions (IMF) $y_i$, where i denotes the index of the respective IMF. By their design, the intrinsic mode functions $y_i$ may be organized in decreasing number of zero-crossings. For each i-th intrinsic mode functions $y_i$ output by the EMD analyzer module an i-th energy $E_i$ may be determined. For this purpose, the respective intrinsic mode functions $y_i$ may be input into an energy determining module (ED) in block 310 configured for determining an intrinsic mode function energy $E_i$ of the respective intrinsic mode function $y_i$.

The i-th intrinsic mode functions $y_i$ may also be input into a frequency determining module (FD) in block 312. The frequency determining module may be configured for determining a frequency $f_i$ of the i-th intrinsic mode functions $y_i$. The frequency $f_i$ may, e.g., be determined using the following approach: In the i-th intrinsic mode functions $y_i$ all up-going and down-going zero-crossings may be detected. Distances between consecutive up-going zero-crossings may be determined as well as distances between down-going zero-crossings. All distances may be stacked and a robust metric, e.g., a median or an alpha-trimmed mean, may be used to determine an average distance over this ensemble of distances. In statistics, the median refers to a value separating a higher half from the lower half of a data sample. The median may be defined as follows: For a data set x of n elements, ordered from smallest to greatest, if n is odd, the median med(x) may be defined as $med(x) = x_{(n+1)/2}$, and if n is even, the median med(x) may be defined as $med(x) = [x_{n/2} + x_{n/2+1}]/2$. The alpha-trimmed mean refers to a mean, which is calculated after discarding p of the elements of a sample at the high and low end, i.e., the smallest p elements and the largest p elements. The alpha-trimmed mean is given by the mean of the remaining $m = n-2p$ elements and may be calculated using the arithmetic mean $\bar{x}$ defined as $\bar{x} = 1/m \cdot \sum_{i=1}^{m} x_i$. Using such a metric may have the beneficial effect, that distances associated with long and short cardiac cycles may be ignored in the metric. The resulting average distance may be translated to an output frequency $f_i$ of the i-th intrinsic mode functions $y_i$. If this output frequency $f_i$ is below the lower limit of the predefined band of heart rates of interest, the iterative EMD process may be terminated as indicated in Fig. 4 by the terminate arrow 314 from the frequency determining module (FD) in block 312 back to the EMDa module in block 308.

A selection module may be used in block 316 to picks, e.g., at most one of the intrinsic mode functions as representative for a PPG signal candidate.

For example, the selection module may be configured to check, if the initial signal $x_0$ is clean enough with respects to the predefined heart rate band of interest or whether the initial signal $x_0$ is too noisy for an accurate extraction of intrinsic mode functions within the predefined heart rate band of interest. This may, e.g., be determined by comparing the initial $E_0$ and pre-processed $E_\varepsilon$, i.e. the total energy of the initial signal $x_0$ with the energy of the components of the initial signal $x_0$ within the predefined heart rate band of interest. For example, only if the ratio of pre-processed energy $E_f$ over initial $E_0$ is sufficiently high, i.e., if the pre-processed energy $E_\varepsilon$ models a significant part of the total initial $E_0$, the input to the EMDa module in block 308 is of sufficient quality. If the pre-processed energy $E_f$ to initial $E_0$ ratio is below a predefined threshold, the method may be terminated for this subset before continuing to the determination of intrinsic mode functions using the EMDa module in block 308 to save computation time. Additionally or alternatively, a ratio of the energy of $E_i$ of the i-th intrinsic mode function $y_i$ over the pre-processed energy $E_f$ may be assessed by the selection module in block 316. If low, i.e., below a pre-defined threshold, the i-th intrinsic mode function $y_i$ may be too small a part of the pre-processed signal $x_f$ to be considered as a viable heartbeat candidate. Additionally, the requirement may be implemented by the selection module in block 316 that the frequency $f_i$ determined for the i-th intrinsic mode function $y_i$ has be located within the predefined heart rate band of interest, in order to consider the i-th intrinsic mode function $y_i$ as a viable heartbeat candidate.

Various ways of combining and/or integrating the aforementioned selection criteria may be implemented by the selection module in block 316. The output of the method for a subset, if not terminated before, may be the intrinsic mode function $y_i$ selected for the respective subset. The output may comprise further features, like frequency $f_i$ and/or energy $E_i$ of the respective intrinsic mode function $y_i$. Furthermore, the output may, e.g., comprise additional metadata associates with the intrinsic mode function $y_i$, like the pre-processed energy $E_f$ of the pre-processed optical signal within the respective subset, the initial energy $E_0$ of the optical signal within the respective subset, the ratio $E_f/E_0$, and/or the ratio $E_i/E_f$. This output data may be used for a clustering of the intrinsic mode functions determined for the subsets and thus of the subsets, to which the respective intrinsic mode functions are assigned.

The pre-processing by the pre-processing module in block 304 may prevent to a large extent intrinsic mode functions $y_i$ with a frequency above the upper limit of the predefined band of heart rates of interest. Together with the feedback of the frequency determination module in block 312, this may prevent the empirical mode decomposition from running a large number of iterations as it may occur, e.g., when noisy signals are input.

The iteration of the empirical mode decomposition may terminate whenever a too low frequency $f_i$ is found, i.e., a frequency below the lower limit of the predefined band of heart rates of interest. This may imply that always an intrinsic mode function may be determined with a too low frequency and thus discarded immediately from further consideration.

A refined approach preventing such a generation of intrinsic mode functions with too low frequencies on a regular basis may, e.g., be implemented in the following form: If a latest determined intrinsic mode function $y_i$ has a frequency $f_i$ close to the lower limit of the predefined band of heart rates of interest, i.e., a frequency $f_i$ within a predefined frequency band above the lower limit of the predefined band of heart rates of interest, the iteration may be determined. In this case it may be assumed that it is likely that the next intrinsic mode function $y_{i+1}$ will have frequency $f_{i+i}$ below the lower limit of the predefined band of heart rates of interest. Additionally or alternatively, if the combined energy of the intrinsic mode functions determined so far is close to the pre-processed energy $E_\varepsilon$, i.e., if a sum of energies of candidate intrinsic mode functions iteratively determined reaching a predefined difference to the pre-processed energy $E_\varepsilon$ of the pre-processed optical signal $x_\varepsilon$ of the respective subset, or, in case of no pre-processing, close to the initial energy $E_0$, then it may be assumed that there are only signal components, intrinsic mode function $y_i$, with small energy left. Both of the aforementioned measures or a suitable combination thereof may be used to implement an early termination criterion.

By the iterative empirical mode decomposition executed by the $EMD_a$ module in block 308, a set of candidate intrinsic mode functions may be determined, from which the intrinsic mode function for the respective subset is selected by the selection module in block 316. For example, from the set of candidate intrinsic mode functions the candidate intrinsic mode function with a largest energy is selected as the intrinsic mode function for describing the optical signal within the respective subset.

Due to its design, i.e., no using of strict periodic/sinusoidal patterns by the EMDa module in block 308 and/or using a robust frequency estimate in the FD module in block 312, the method may be able to handle deviant cardiac cycles. Due to the pre-processing in block 304 and/or the frequency estimate feedback 314, a low-cost computational design may be implemented. Due to the optional additional parameter extraction of parameters, like $E_0$, $E_f$, $E_i$, and/or $f_i$, the selection rules may be implemented by the selection module 316, which may conceptually agree and may be at least equivalent with those parameters used in an FFT-peak signal determination, where a signal strength of a dominant frequency may be used in combination with in- and out-of-band energies.

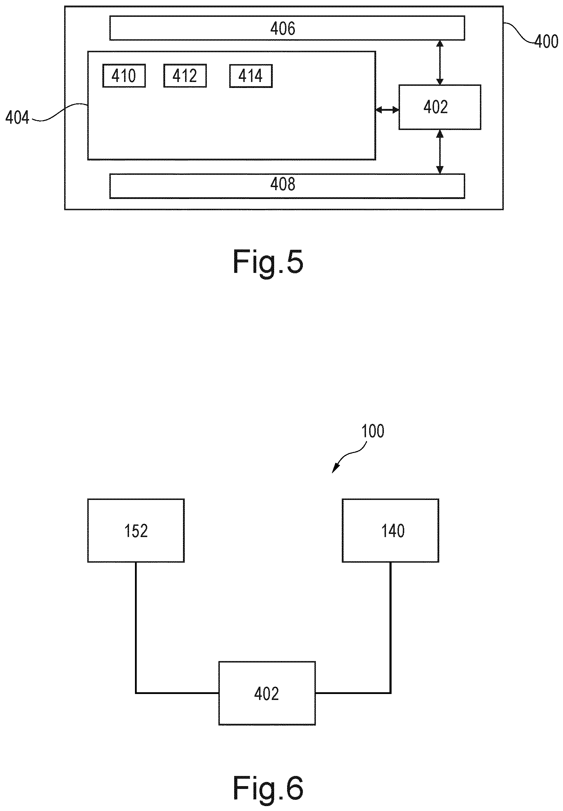

Fig. 5 illustrates an exemplary computational system 400 for determining a region of interest for extracting a photoplethysmography signal of a subject within optical images. The computational system 400 is shown as comprising a computational component 402. The computational component 402 is intended to represent one or more processors or processing cores or other computational elements. The computational component 402 is shown as being connected to a hardware interface 406 and a memory 404. The hardware interface 406 may enable the computational component 402 to exchange commands and data with other components, e.g., an optical sensor. The hardware interface 406 may, e.g., enable the computational component 402 to control the optical sensor to acquire the optical imaging data, e.g., a sequence of optical images 412. The hardware interface 406 may for example further enable the computational component 402 to control a medical imaging system.

The computational system 404 is further shown as being connected to a user interface 408 which may for example enable an operator to control and operate the computational system 400 and via the computational system 400 an optical sensor and/or a medical imaging system. The user interface 408 may, e.g., comprise an output and/or input device enabling a user to interact with the computer 400. The output device may, e.g., comprise a display device configured for displaying images of the sequence of images 412, a region of interest 414 determined using the sequence of images 412, a PPG signal extracted from the region of interest 414 and/or medical images reconstructed using medical imaging data acquired with a medical imaging system. The input device may, e.g., comprise a keyboard and/or a mouse enabling the user to insert control commands for controlling the computational system 400 and via the computational system 400 the optical sensor and/or the medical imaging system.

The memory 404 is shown as containing machine-executable instructions 410. The machine-executable instructions 410 enable the computational component 402 to perform controlling tasks, such as controlling the optical sensor and/or the medical imaging system, to perform numerical tasks, as well as performing various signal data processing tasks, like determining a region of interest 414 and/or extracting a PPG signal. The machine-executable instructions 410 may, e.g., enable the computational component 402 and thus the computational system 400 to determine a region of interest for extracting a PPG signal according to any of Fig. 1, 3 and 4 and/or to determining and using a region of interest for extracting a PPG signal according to Fig. 2. For example, the instructions 410 may, e.g., enable the computational component 402 and thus the computational system 400 to control a medical imaging system.

The memory 404 is further shown as containing a sequence of optical images 412 received, e.g., from an optical sensor. The optical images of sequence 412 may comprise optical imaging data of at least a part of a tissue of interest of a subject, which is of interest for the PPG signal extraction.

The memory 404 is further shown as containing a definition of a region of interest 414 determined using the sequence of optical images 412.

Fig. 6 illustrates an exemplary medical imaging system 100 comprising the computational system 400 of Fig. 5. The medical imaging system 100 further comprises a medical sensor system 152 configured for acquiring medical imaging data of a subject and an optical sensor 140 configured for acquiring optical images of the subject. The computational system 400 may be configured to control the medical sensor system 152 and the optical sensor 140. In addition, the medical imaging system 100 may, e.g., comprise an illumination source for illuminating the subject, when acquiring the optical images using the optical sensor 140.

The computational system 400 may control the optical sensor 140 to acquire a sequence of optical images, which is received by the computational system 400. In addition, the computational system 400 may control the illumination source for illuminating the subject, when acquiring the optical images using the optical sensor 140. The computational system 400 may process the sequence of optical images and determine a region of interest within the images of the sequence of optical images. For this purpose, an empirical mode decomposition may be used to determine intrinsic mode functions indicative of temporal signal dynamics of optical signals within subsets of pixels of the images of the sequence of optical images. The region of interest may be used for extracting a PPG signal of the subject within optical images. The computational system 400 may use the extracted PPG signal for controlling an acquiring of medical imaging data of the subject using the medical sensor system 152.

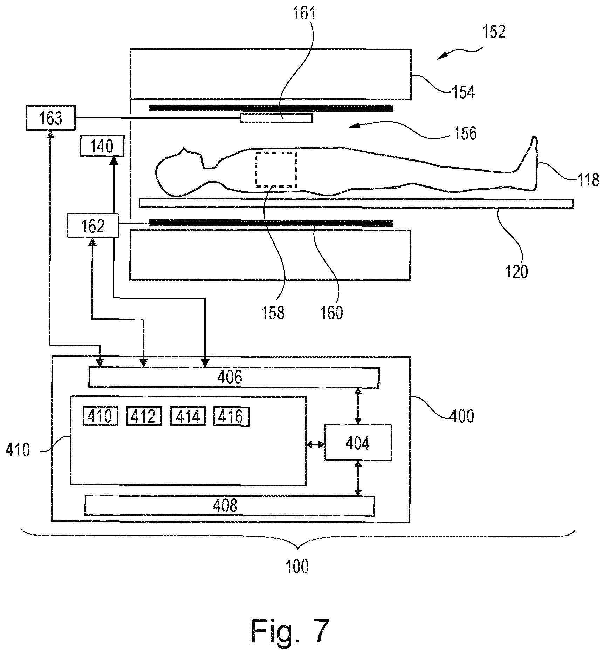

Fig. 7 illustrates an exemplary medical imaging system 100 in form of an MRI system comprising a computing device 400. The computational system 400 is configured for determining a region of interest for extracting a PPG signal of a subject 118 within optical images. The computational system 400 of Fig. 7 is, e.g., the computational system 400 of Fig. 5.

The medical imaging system 100, e.g., comprises an optical sensor 140 configured acquiring optical imaging data of the subject 118, e.g., in form of sequences of optical images. The optical sensor 140 is controlled by the computational system 400. In addition, the medical imaging system 100 may, e.g., comprise an illumination source for illuminating the subject, when acquiring the optical images using the optical sensor 140. The medical imaging system 100, e.g., comprises an exemplary medical sensor system 152 in form of a magnetic resonance imaging sensor system.

Alternatively, the medical imaging system 100 may, e.g., comprise a computed tomography system, a positron emission tomography system, or a single-photon emission computed tomography system.

A subject 118 is shown as being supported by a table 120. The MRI sensor system 152 is controlled by the computer 400. The MRI sensor system 152 comprises a magnet 154. The magnet 154 is a superconducting cylindrical type magnet with a bore 156 through it. The use of different types of magnets is also possible. For instance, it is also possible to use both a split cylindrical magnet and a so-called open magnet.

Within the bore 156 of the cylindrical magnet 154 there is an imaging zone 158 of MRI sensor system 152. In the imaging zone 158, the magnetic field is, e.g., strong and uniform enough to perform magnetic resonance imaging.

Within the bore 156 of the magnet there is also a set of magnetic field gradient coils 160 which is used for acquisition of preliminary magnetic resonance data to spatially encode magnetic spins within the bore 156 of magnet 154. The magnetic field gradient coils 160 are connected to a magnetic field gradient coil power supply 162. The magnetic field gradient coils 160 are intended to be representative. Typically, magnetic field gradient coils 160 contain three separate sets of coils for spatially encoding in three orthogonal spatial directions. The magnetic field gradient power supply 162 supplies current to the magnetic field gradient coils 160. The current supplied to the magnetic field gradient coils 160 is controlled as a function of time and may be ramped or pulsed.

Furthermore, the MRI sensor system 152 may comprise an RF transmitter coil 161 for manipulating the orientations of magnetic spins. The RF transmitter coil 161 may also be referred to as a transmitter antenna. The RF transmitter coil 161 is connected to an RF transmitter 163. It is understood that the RF transmitter coil 161 and the RF transmitter 163 are representative. The RF transmitter coil 161 may have multiple transmitter elements and the RF transmitter 163 may have multiple transmitter channels.

The transmitter 163, the gradient controller 162, and the optical sensor 140 are shown as being connected to the hardware interface 406 of the computer 400. The computational system 400 is intended to represent one or more computational or computing devices. The computational system 400 is configured for acquiring medical imaging data 416 as part of a control system of the medical imaging system 100, e.g., MRI images using the MRI sensor system 152. The computational system 400 is further configured for controlling the optical sensor 140 for acquiring optical images 412. These optical images may be used for extracting a PPG signal from a region of interest 414 determined within the optical images 412. The PPG signal may be used by the computational system 400 to control the MRI sensor system 152 for acquiring the MRI imaging data 416.

For example, the optical sensor 140 may be integrated in, or mounted on, a flange of the MR bore 156, e.g., such that the usable free bore diameter is not affected or only minimally reduced, and/or to avoid or minimize interference with the operation of the MR system. For example, optional illuminating sources may also be provided, e.g., in or on this.

The system may comprise a display 408 to display images of the inside of the bore 156 acquired by the optical sensor 140. This enables an operator to visually monitor the subject in the bore 156.

Respiratory and/or cardiac phase information and/or more generic information indicative of motion, e.g. the PPG signal extracted and/or information derived therefrom and/or enriched thereby, may be used to correct the acquired magnetic resonance signals for motion and/or apply motion corrections to the reconstructed magnetic resonance images 416. For example, a cardiac trigger signal may be determined based on the extracted PPG signal.

The PPG signal may be used to gate a data acquisition by the medical imaging system 100 system. Additionally or alternatively, the PPG signal may be used to sort, collate, select and/or annotate reconstructed magnetic resonance images 416, e.g. to show extracted PPG information or information derived therefrom, such as a cardiac phase, alongside the magnetic resonance images 416 acquired at substantially the corresponding time.

It is understood that one or more of the aforementioned examples may be combined as long as the combined embodiments are not mutually exclusive.

As will be appreciated by one skilled in the art, aspects of the present invention may be embodied as an apparatus, method, or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid-state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a computational system. 'Computer storage' or 'storage' is a further example of a computer-readable storage medium. Computer storage is any non-volatile computer-readable storage medium. In some embodiments computer storage may also be computer memory or vice versa.

A 'computational system' as used herein refers to an electronic component comprising a computational component which is able to execute a program or machine executable instruction or computer executable code. References to the computational system comprising the example of "a computational system" should be interpreted as possibly containing more than one computational component or processing core. The computational system may for instance be a multi-core processor.

A computational system may also refer to a collection of computational components within a single computer system or distributed amongst multiple computer systems. The term computational system should also be interpreted to possibly refer to a collection or network of computing devices each comprising a processor or computational component. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further understood that, when not mutually exclusive, combinations of blocks in different flowcharts, illustrations, and/or block diagrams may be combined. These computer program instructions may be provided to a computational system of a general-purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen, Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

REFERENCE SIGNS LIST

| | |
|---|---|
| 100 | medical imaging system |
| 118 | subject |
| 120 | table |
| 140 | optical sensor |
| 152 | medical sensor system |
| 154 | magnet |
| 156 | bore of magnet |
| 158 | imaging zone |
| 160 | magnetic field gradient coils |
| 161 | radio-frequency transmitter coil |
| 162 | magnetic field gradient coil power supply |
| 163 | transmitter |
| 400 | computational system |

| 402 | computational component |
|---|---|
| 404 | memory |
| 406 | hardware interface |
| 408 | user interface |
| 410 | machine readable instructions |
| 412 | sequence of optical images |
| 414 | region of interest |
| 416 | medical images |
| $E_0$ | initial energy |
| $E\varepsilon$ | pre-processed energy |
| $E_i$ | energy of i-th intrinsic mode function |
| ED | energy determining module |
| $EMD_a$ | empirical mode decomposition analyzer module |
| FD | frequency determining module |
| SPP | signal pre-processing |
| $x_0$ | intial optical signal |
| $x_f$ | pre-processed signal |
| $y_i$ | i-th intrinsic mode function |

**Claims**

1. A computational system (400) for determining a region of interest (414) for extracting a photoplethysmography signal of a subject (118) within optical images, wherein the computational system (400) comprises a processor (402) and a memory (404) storing machine executable instructions (410), wherein an execution of the machine executable instructions (410) using the processor (402) causes the computational system (400) to:

   - receive a sequence of optical images (412), the optical images comprising optical imaging data of at least a part of a tissue of interest of the subject (118), which is of interest for the photoplethysmography signal extraction;
   - for the sequence of optical images (412), divide a respective image region comprised by each optical image of the sequence (412) into a plurality of subsets of pixels;
   - determine for at least some of the subsets dynamic characteristics of optical signals (xo) comprised by the respective subsets, wherein each of the dynamic characteristics is described by an intrinsic mode function ($y_i$), wherein the respective intrinsic mode function ($y_i$) is indicative of a temporal signal dynamic of the optical signal (xo) within the respective subset over the sequence of optical images (412);
   - cluster the plurality of subsets, using the determined dynamic characteristics, to group the subsets of pixels into clusters with similar signal dynamics;
   - select a cluster from the resulting clusters comprising a cluster characteristic satisfying a pre-defined selection criterium; and
   - identify the pixels comprised by the selected cluster as the region of interest (414) of the optical images for extracting the photoplethysmography signal of the subject (118).

2. The computational system (400) of claim 1, wherein the determining of the intrinsic mode ($y_i$) functions further comprises a pre-processing of the optical signals ($x_0$) comprised by the plurality of subsets, wherein the pre-processing comprises removing signal components of the optical signals ($x_0$) with frequencies above an upper limit of a predefined band of heart rates of interest.

3. The computational system (400) of claim 2, wherein a selecting of the at least some subsets comprises per subset of the plurality of subsets a determining of a ratio of a pre-processed energy ($E_f$) of the pre-processed optical signal ($x_f$) of the respective subset to an initial energy ($E_0$) of the optical signal (xo) before the pre-processing, the selected subsets from the plurality of subsets being subsets for which the ratio reaches a first predefined threshold.

4. The computational system (400) of any of the previous claims, wherein the empirical mode decomposition for determining the intrinsic mode functions ($y_i$) comprises for each of the subsets an iteratively determining of one or more candidate intrinsic mode functions ($y_i$), from which the intrinsic mode function ($y_i$) is selected.

5. The computational system (400) of claim 4, wherein from the one or more candidate intrinsic mode functions ($y_i$) the candidate intrinsic mode function ($y_i$) with a largest energy is selected as the intrinsic mode function ($y_i$).

6. The computational system (400) of any of claims 4 to 5, wherein the determining of the candidate intrinsic mode functions ($y_i$) comprises per candidate intrinsic mode function ($y_i$) determining a ratio of energy ($E_i$) of the respective candidate intrinsic mode function ($y_i$) to a pre-processed energy ($E_f$) of the pre-processed optical signal ($x_f$) of the subset, for which the respective candidate intrinsic mode function (y;) is determined, wherein candidate intrinsic mode functions ($y_i$), for which the ratio reaches a predefined second threshold, are discarded for the selection of the intrinsic mode function ($y_i$).

7. The computational system (400) of any of claims 4 to 6, wherein the determining of the candidate intrinsic mode functions ($y_i$) further comprises determining frequencies (f;) of the candidate intrinsic mode functions ($y_i$), wherein candidate intrinsic mode functions ($y_i$) with frequencies (f;) above the upper limit of the predefined band of heart rates of interest and/or candidate intrinsic mode functions ($y_i$) with frequen-

cies ($f_i$) below a lower limit of the predefined band of heart rates of interest are discarded for the selection of the intrinsic mode function ($y_i$).

8. The computational system (400) of any of claims 4 to 7, wherein the iterative determining of candidate intrinsic mode functions ($y_i$) is terminated upon reaching a termination criterium.

9. The computational system (400) of claim 8, wherein the termination criterium is any one of the following: a determining of an intrinsic mode function ($y_i$) with a frequency ($f_i$) below the lower limit of the predefined band of heart rates of interest; a determining of an intrinsic mode function ($y_i$) with a frequency ($f_i$) within a predefined frequency band above the lower limit of the predefined band of heart rates of interest; and a sum of energies of the candidate intrinsic mode functions ($y_i$) iteratively determined reaching a predefined difference to the pre-processed energy ($E_f$) of the pre-processed optical signal ($x_f$) of the respective subset.

10. The computational system (400) of claim 1, wherein the execution of the machine executable instructions (410) further causes the computational system (400) to discard from the determining of the intrinsic mode functions ($y_i$) such subsets, which satisfy one or more predefined signal criteria for one or more signal characteristics of the optical signals (xo), the signal characteristics being determined on a subset-per-subset basis, the one or more signal criteria comprising one or more of the following: a mean signal intensity per subset, a spread of signal intensity per subset, and at least one value indicative of a motion associated with the respective subset.

11. The computational system (400) of any of the previous claims, wherein the execution of the machine executable instructions (410) further causes the computational system (400) to extract the photoplethysmography signal from the region of interest (414) identified within the optical images.

12. The computational system (400) of claim 11, wherein the execution of the machine executable instructions (410) further causes the computational system (400) to use the extracted photoplethysmography signal for controlling an acquiring of medical imaging data of the subject (118) using a medical imaging system (100).

13. A medical imaging system (100), the medical imaging system (100) comprising:

   - a medical sensor system (152) configured for acquiring medical imaging data of a subject (118),

   - an optical sensor (140) configured for acquiring optical images of the subject (118),
   - the computational system (400) of any of the previous claims.

14. A method for determining a region of interest (414) for extracting a photoplethysmography signal of a subject (118) within optical images, wherein the method comprises:

   - receiving a sequence of optical images (412), the optical images comprising optical imaging data of at least a part of a tissue of interest of the subject (118), which is of interest for the photoplethysmography signal extraction;
   - for the sequence of optical images (412), dividing a respective image region comprised by each optical image of the sequence (412) into a plurality of subsets of pixels;
   - determining for at least some of the subsets dynamic characteristics of optical signals (xo) comprised by the respective subsets, wherein each of the dynamic characteristics is described by an intrinsic mode function ($y_i$), wherein the respective intrinsic mode function ($y_i$) is indicative of a temporal signal dynamic of the optical signal (xo) within the respective subset over the sequence of optical images (412);
   - clustering the plurality of subsets, using the determined dynamic characteristics, to group the subsets of pixels into clusters with similar signal dynamics;
   - selecting a cluster from the resulting clusters comprising a cluster characteristic satisfying a pre-defined criterium; and
   - identifying the pixels comprised by the selected cluster as the region of interest (414) of the optical images for extracting the photoplethysmography signal of the subject (118).

15. A computer program comprising machine executable instructions (410) for determining a region of interest (414) for extracting a photoplethysmography signal of a subject (118) within optical images, wherein an execution of the machine executable instructions (410) using a processor (402) of a computational system (400) causes the computational system (400) to:

   - receive a sequence of optical images (412), the optical images comprising optical imaging data of at least a part of a tissue of interest of the subject (118), which is of interest for the photoplethysmography signal extraction;
   - for the sequence of optical images (412), divide a respective image region comprised by each optical image of the sequence (412) into a plurality of subsets of pixels;

- determine for at least some of the subsets dynamic characteristics of optical signals ($x_0$) comprised by the respective subsets, wherein each of the dynamic characteristics is described by an intrinsic mode function ($y_i$) wherein the respective intrinsic mode function ($y_i$) is indicative of a temporal signal dynamic of the optical signal (xo) within the respective subset over the sequence of optical images (412);

- cluster the plurality of subsets, using the determined dynamic characteristics, to group the subsets of pixels into clusters with similar signal dynamics;

- select a cluster from the resulting clusters comprising a cluster characteristic satisfying a pre-defined criterium; and

- identify the pixels comprised by the selected cluster as the region of interest (414) of the optical images for extracting the photoplethysmography signal of the subject (118).

```
┌─────────┐
│   200   │
└─────────┘
     │
     ▼
┌─────────┐
│   202   │
└─────────┘
     │
     ▼
┌─────────┐
│   204   │
└─────────┘
     │
     ▼
┌─────────┐
│   206   │
└─────────┘
     │
     ▼
┌─────────┐
│   208   │
└─────────┘
     │
     ▼
┌─────────┐
│   210   │
└─────────┘
     │
     ▼
┌─────────┐
│   212   │
└─────────┘
```

Fig. 1

Fig. 2

Fig. 3

EP 4 606 297 A1

Fig. 4

## Fig.5

## Fig.6

Fig. 7

Europäisches
Patentamt

European
Patent Office

Office européen
des brevets

# EUROPEAN SEARCH REPORT

**Application Number**

EP 24 15 8668

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SONG RENCHENG ET AL: "Remote Photoplethysmography With an EEMD-MCCA Method Robust Against Spatially Uneven Illuminations", IEEE SENSORS JOURNAL, IEEE, USA, vol. 21, no. 12, 22 March 2021 (2021-03-22), pages 13484-13494, XP011860815, ISSN: 1530-437X, DOI: 10.1109/JSEN.2021.3067770 [retrieved on 2021-06-11] * Section III-IV; page 13486 - page 13489; figures 1-4 * | 1,2,4,5, 7,8,10, 11,13-15 | INV. A61B5/00 A61B5/024 |
| A | CHENG JUAN ET AL: "Exploring the feasibility of seamless remote heart rate measurement using multiple synchronized cameras", MULTIMEDIA TOOLS AND APPLICATIONS, KLUWER ACADEMIC PUBLISHERS, BOSTON, US, vol. 79, no. 31-32, 5 June 2020 (2020-06-05), pages 23023-23043, XP037220658, ISSN: 1380-7501, DOI: 10.1007/S11042-020-09075-2 [retrieved on 2020-06-05] * page 23026 - page 23033; figures 1-3 * | 1-15 | |
| | ----- | | TECHNICAL FIELDS SEARCHED (IPC) |
| | -/-- | | A61B |

The present search report has been drawn up for all claims

| Place of search | Date of completion of the search | Examiner |
|---|---|---|
| The Hague | 2 July 2024 | Hirsch, Arthur |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

EP 24 15 8668

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | XUXUE SUN ET AL: "Robust heart beat detection from photoplethysmography interlaced with motion artifacts based on Empirical Mode Decomposition", BIOMEDICAL AND HEALTH INFORMATICS (BHI), 2012 IEEE-EMBS INTERNATIONAL CONFERENCE ON, IEEE, 5 January 2012 (2012-01-05), pages 775-778, XP032449184, DOI: 10.1109/BHI.2012.6211698 ISBN: 978-1-4577-2176-2 * section II II. EMD-BASED ROBU ST HEART RATE EXTRACTION; paragraph [0775] - paragraph [0778] * ----- | 1-15 | |
| A | ZHANG YUZHONG ET AL: "Illumination variation-resistant video-based heart rate monitoring using LAB color space", OPTICS AND LASERS IN ENGINEERING, ELSEVIER, AMSTERDAM, NL, vol. 136, 8 August 2020 (2020-08-08), XP086326895, ISSN: 0143-8166, DOI: 10.1016/J.OPTLASENG.2020.106328 [retrieved on 2020-08-08] * Section II; page 1 - page 7; figures 1-6 * ----- | 1-15 | **TECHNICAL FIELDS SEARCHED (IPC)** |

The present search report has been drawn up for all claims

| Place of search | Date of completion of the search | Examiner |
|---|---|---|
| The Hague | 2 July 2024 | Hirsch, Arthur |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................

& : member of the same patent family, corresponding document

EPO FORM 1503 03.82 (P04C01)